(19) Europäisches Patentamt
European Patent Office
Office européen des brevets

(11) Numéro de publication: **0 275 728 B1**

(12) **FASCICULE DE BREVET EUROPEEN**

(45) Date de publication de fascicule du brevet: **29.05.91**

(51) Int. Cl.5: **C07J 31/00**, C07J 9/00, C07J 7/00, C07J 41/00

(21) Numéro de dépôt: **87402739.4**

(22) Date de dépôt: **03.12.87**

(54) **Nouveaux produits stéroîdes comportant, en position 23, un radical sulfinyle, leur procédé de préparation, leur application à la préparation de produits de la série des 20-cétoprégnanes et des intermédiaires de cette application.**

(30) Priorité: **05.12.86 FR 8617050**

(43) Date de publication de la demande: **27.07.88 Bulletin 88/30**

(45) Mention de la délivrance du brevet: **29.05.91 Bulletin 91/22**

(84) Etats contractants désignés: **AT BE CH DE ES FR GB GR IT LI LU NL SE**

(56) Documents cités:
**US-A- 2 705 232**
**US-A- 4 500 460**

**JOURNAL OF THE AMERICAN CHEMICAL SOCIETY, vol. 74, no. 23, 5 décembre 1952, pages 5814-5818, Washington, D.C., US; R.B. TURNER et al.: "Steroids derived from bile acids. XIX. Barbier-Wieland degradation in the 11-keto series"**

(73) Titulaire: **ROUSSEL-UCLAF**
**35, boulevard des Invalides**
**F-75007 Paris(FR)**

(72) Inventeur: **Vivat, Michel**
**14, Chemin de l'Autostrade**
**F-77400 Lagny-Sur-Marne(FR)**
Inventeur: **Buendia, Jean**
**3bis, Impasse Emilie**
**F-94170 LE Perreux-Sur-Marne(FR)**

(74) Mandataire: **Bourgouin, André et al**
**Département des Brevets ROUSSEL UCLAF**
**111, route de Noisy B.P. no 9**
**F-93230 Romainville(FR)**

**EP 0 275 728 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux produits stéroïdes comportant en position 23 un radical sulfinyle, leur procédé de préparation, leur application à la préparation de produits de la série des 20-céto pregnane et des intermédiaires de cette application.

La présente invention a pour objet les produits de formule générale (I) :

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

$$-N\begin{matrix} R_3 \\ R_4 \end{matrix}$$

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote

auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone.

Lorsque les cycles A, B, C et D portent une ou plusieurs doubles liaisons il s'agit de préférence de doubles liaisons en 1(2), 4(5), 5(6) ou 9(11) ou d'un système de doubles liaisons conjuguées en 3(4) et 5(6) ou en 4(5) et 6(7) ou d'un système aromatique de trois doubles liaisons 1, 3, 5 ou d'un système de trois doubles liaisons 1(2), 4(5), 6(7). On utilise cependant de préférence des produits de comportant pas de double liaison.

Lorsque les cycles A, B, C et D sont substitués par une ou plusieurs fonctions hydroxyles, il s'agit de préférence d'une ou plusieurs fonctions hydroxyles en 3, 6, 7, 11 et/ou 12.

Lorsque les cycles A, B, C et D sont substitués par une ou plusieurs fonctions cétones, il s'agit de préférence d'une fonction cétone en 3, en 7, en 11 ou en 12.

Lorsque les cycles A, B, C et D sont substitués par un ou plusieurs atomes d'halogènes, il s'agit de préférence d'un atome de fluor, de chlore ou de brome, en position 6 ou 9 alpha par exemple.

Lorsque les cycles A, B, C et D sont substitués par un ou plusieurs radicaux alkyles, il s'agit de préférence du radical méthyle ou éthyle en 2, en 6, en 7, en 16 alpha ou en 16 béta.

Lorsque les cycles A, B, C et D sont substitués par un ou plusieurs radicaux alkyloxy, il s'agit de préférence d'un radical méthoxy ou éthoxy en 3 ou 11 béta.

Les groupements hydroxyles peuvent être protégés selon les modes habituels connus dans la littérature. On peut par exemple citer les groupements acétonides, les carbonates cycliques, les orthoesters, les sulfites cycliques, l'éther formé avec le tétrahydropyrannyle, le groupement trityle, benzyle, les radicaux acyles, tels qu'acétyle, succinyle ou formyle.

Les groupements cétoniques peuvent de même être protégés par les groupements protecteurs classiques, tels que les cétals, plus spécialement l'éthylène cétal, les thioacétals, les hémithioacétals, les éthers d'énols, les acétates d'énols, les énamines, les oximes.

On préfère cependant les groupements cétals et spécialement l'éthylène cétal pour protéger les

groupements cétoniques. Lorsque les produits de formule I comportent un groupement cétonique en position 3, ce groupement est très préférentiellement protégé.

R peut représenter un atome d'halogène, de préférence un atome de chlore ou de brome, R peut également représenter un radical alkoxy tel que méthoxy ou éthoxy de préférence mais également propyloxy, isopropyloxy, butyloxy, sec-butyloxy, tert-butyloxy, pentyloxy, hexyloxy, R peut également représenter un radical benzyloxy, phényléthyloxy.

Les radicaux $R_3$ et $R_4$ identiques ou différents peuvent représenter un atome d'hydrogène ou des radicaux méthyle, éthyle, propyle, isopropyle, butyle, sec-butyle, tert-butyle, pentyle, hexyle, benzyle, soit $R_3$ et $R_4$ forment ensemble avec l'atome d'azote auquel ils sont liés un radical morpholine, pipéridine, pyrrolidine. R peut également représenter un radical méthylthio ou éthylthio ou un radical alkylthio dérivé des radicaux alkyle ou alkoxy indiqués ci-dessus. R peut également représenter un radical phénylthio ou benzylthio.

L'invention a plus particulièrement pour objet les produits de formule générale I telle que définie ci-dessus dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégée, en position 6, 7, 11 et 12 et les fonctions cétones éventuellement protégée, en position 7, 11 et 12 et R représente un atome d'halogène, un radical hydroxy, un radical alkoxy ayant au plus 4 atomes de carbone ou un radical :

$$-N\begin{matrix} R'_3 \\ R'_4 \end{matrix}$$

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pipéridino, pyrrolidino ou morpholino et plus spécialement les produits de formule générale I telle que définie ci-dessus dans laquelle les noyaux A, B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un atome de chlore ou un radical hydroxyle.

Parmi les produits préférés une sous-famille de produits particulièrement avantageux est constituée par les produits de formule générale I telle que définie ci-dessus, dans laquelle les noyaux A, B, C, D portent en position 3 un radical hydroxyle éventuellement protégé et, éventuellement, une ou plusieurs autres fonctions choisies parmi les fonctions hydroxyles éventuellement protégées en position 6, 7 ou 12 et les fonctions cétones éventuellement protégées, en position 7, 11 ou 12.

Dans cette dernière famille, on peut citer les produits comportant, comme squelette les cycles A, B, C, D, les produits dérivés des acides biliaires naturels ou hémi-synthétiques. Ces produits peuvent être énumérés par le tableau suivant :


R12
CH3
CH3
HO
R7
R6
COR'
S
O


dans laquelle R' représente un atome de chlore ou un radical hydroxyle et $R_6$, $R_7$ et $R_{12}$ ont les significations suivantes :

| $R_6$ | $R_7$ | $R_{12}$ |
|---|---|---|
| H | OH alpha | OH alpha |
| H | OH béta | OH alpha |
| H | H | H |
| H | H | OH alpha |
| H | OH alpha | H |
| OH alpha | H | H |
| H | OH béta | H |
| OH alpha | OH alpha | H |
| OH béta | OH alpha | H |
| OH béta | OH béta | H |
| H | OH alpha | OH alpha |
| H | H | OH alpha |

Dans ces produits, le ou les radicaux hydroxyles peuvent également être protégés notamment le radical hydroxyle en position 3. Le groupement protecteur préféré peut être le groupement acétyle ou le groupement formyle.

Parmi les produits comportant une ou plusieurs fonctions cétones, on préfère les produits suivants :

$CH_3$ $CH_3$ A B C D COR' S O

dans lesquels R' représente un radical hydroxyle et les substituants en position 3, 7, 11 et 12 ont les significations suivantes :

- 3 cétone protégée,
- 3 OH alpha, 7 céto, 12 OH alpha,
- 3 OH alpha, 11 céto
- 3 OH alpha, 7 OH alpha, 12 céto
- 3 OH alpha, 7 céto
- 3 OH alpha, 7 OH béta, 12 céto
- 3 OH, 11 céto, 12 OH
- 3 OH, 11 céto.

Bien entendu, comme précédemment, les radicaux hydroxyles peuvent être protégés. Il en est de même des radicaux cétoniques en position 7 ou 12. Le groupement protecteur préféré du groupement cétonique est un cétal cyclique ou non cyclique.

Les produits préférés sont les produits décrits ci-après dans les exemples, et notamment :

- l'acide 3 alpha formyloxy 11-oxo 23-sulfinyl 5 béta-cholan-24-oïque.
- l'acide 3 alpha hydroxy 11-oxo 23-sulfinyl 5 béta-cholan-24-oïque.

La présente invention a également pour objet un procédé de préparation des produits de formule générale I telle que définie ci-dessus, caractérisé en ce que l'on traite un produit de formule II :

(II)

dans laquelle A, B, C, D, $R_1$ et $R_2$ ont la signification indiquée ci-dessus d'abord par un agent de formation d'un halogénure d'acide, ensuite par une base tertiaire puis par le chlorure de thionyle et enfin éventuellement par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée ci-dessus un alkylthiol, un arylthiol ou un aralkylthiol pour obtenir un produit de formule I attendu.

Dans un mode préféré d'exécution du procédé ci-dessus l'agent de formation d'un halogénure d'acide que l'on utilise est choisi notamment parmi le chlorure de thionyle, le chlorure ou le bromure d'oxalyle. Parmi ces réactifs, le chlorure de thionyle est le réactif préféré.

La base tertiaire que l'on utilise de préférence est choisie parmi la triéthylamine, la méthyl éthyl pyridine, la pyridine, le diazabicyclooctène, le diazabicyclononène, le diazabicycloundecane, plus particulièrement la triéthylamine ou la pyridine.

L'alcanol ou l'aralcanol que l'on utilise de préférence est le méthanol, l'éthanol ou l'alcool benzylique; l'amine primaire ou secondaire que l'on peut utiliser est choisie parmi la méthyl ou éthylamine, la diéthylamine, la morpholine, la pipéridine, la pyrrolidine, on peut bien entendu utiliser un thiol correspondant.

L'alkylthiol, l'arylthiol ou l'aralkylthiol que l'on peut utiliser est choisi de préférence parmi le méthanethiol, l'éthanethiol ou l'alcool thiobenzylique.

Les réactions énoncées ci-dessus peuvent de préférence être effectuées dans un solvant ou un mélange de solvants peu ou non miscibles à l'eau tels que le chlorure de méthylène, le chloroforme.

Bien entendu, les réactions classiques de blocage ou de déblocage des groupements fonctionnels que peuvent comporter les cycles A, B, C et D peuvent être effectuées au départ de la synthèse, sur les produits de formule II ou sur les produits de formule I obtenus. On peut par exemple soumettre les produits de formule I dans laquelle le cycle A comporte en position 3, un radical hydroxyle protégé par un radical acyle tel que acétyle ou formyle à une réaction classique de saponification pour obtenir le produit correspondant dans lequel le cycle A comporte un radical hydroxyle libre. On opère selon les méthodes usuelles par action, par exemple, d'une base telle que la soude, la potasse ou le carbonate de potassium dans un solvant tel que le méthanol, le chlorure de méthylène, l'eau ou un mélange de ces solvants.

La présente invention a également pour objet une application des produits de formule I telle que définie ci-dessus à la préparation des produits de formule IV :

(IV)

dans laquelle A, B, C, D, $R_1$ et $R_2$ ont la signification indiquée ci-dessus caractérisée en ce que, lorsque R représente un radical hydroxyle, on traite éventuellement un produit de formule I par un agent de formation d'un halogénure d'acide pour obtenir un produit de formule I dans laquelle R représente un atome

d'halogène et en ce que l'on traite un produit de formule I dans lequel R a la signification indiquée ci-dessus par un réactif d'halogénation et lorsque R représente un atome d'halogène traite éventuellement le produit intermédiaire obtenu par l'eau, un alcanol, un aralcanol, une amine primaire ou secondaire de formule :

$$H{-}N\begin{matrix}\diagup R_3\\ \diagdown R_4\end{matrix}$$

dans laquelle $R_3$ et $R_4$ ont la signification indiquée ci-dessus, un alkylthiol, un arylthiol ou un aralkylthiol pour obtenir un produit de formule III :

(III)

produit de formule III que l'on soumet d'abord à un agent de déshydrohalogénation puis à un agent de coupure oxydante pour obtenir un produit de formule IV attendu.

L'agent de formation éventuelle d'un halogénure d'acide que l'on utilise est choisi dans la liste indiquée ci-dessus, de préférence le chlorure de thionyle.

Le réactif d'halogénation que l'on utilise est un halogène tel que le brome ou un réactif d'halogénation tel que le chlorure de sulfuryle.

On obtient alors, lorsque R représente un atome d'halogène, un produit intermédiaire de formule $III_A$ :

$(III_A)$

dans laquelle Hal et $Hal_1$ représentent un atome d'halogène sur lequel on fait agir éventuellement et dans les mêmes conditions qu'indiquées ci-dessus, l'eau, un alcanol, un aralcanol, une amine primaire ou secondaire de formule :

$$H{-}N\begin{matrix}\diagup R_3\\ \diagdown R_4\end{matrix}$$

un alkylthiol, un arylthiol, un aralkylthiol pour obtenir un produit de formule III tel qu'indiqué ci-dessus.

L'agent de déshydrohalogénation que l'on fait agir sur le produit de formule III est de préférence un agent basique fort tel que le Triton B

$$(C_6H_5CH_2\overset{CH_3}{\underset{CH_3}{N^{\oplus}}}-CH_3\ \ OH^{\ominus}),$$

un alcoolate de métal alcalin tel que l'éthanolate de sodium ou de potassium, le tert-butylate de potassium, un amidure de sodium ou de potassium. On pourrait également envisager d'utiliser une base telle que la soude ou la potasse au reflux dans un alcanol tel que le méthanol ou l'éthanol ou le glyme. On peut enfin envisager d'utiliser une résine basique telle que l'Amberlite.

L'agent de coupure oxydante que l'on peut utiliser pour obtenir en fin de synthèse les produits de formule IV attendus est choisi de préférence parmi l'ozone et un oxydant tel que l'oxyde de ruthénium ou l'oxyde de manganèse.

D'après les constatations de la demanderesse, l'action d'un réactif de déshydrohalogénation sur les produits de formule III donne naissance à un mélange de produits répondant aux formules suivantes :

(III1) ; (III2) ; (III3)

qui sont ensuite, pour les produits de formules $III_1$ et $III_2$ susceptibles de conduire, après coupure oxydante aux produits de formule IV. Le procédé de la demanderesse ne conduit qu'en faible quantité au produit de formule $III_3$ non susceptible de conduire au produit final recherché.

La présente invention a également pour objet un procédé de préparation des produits de formule IV tels que définis ci-dessus caractérisé en ce que l'on fait agir sur un produit de formule II un agent de formation d'un halogénure d'acide, une base tertiaire puis le chlorure de thionyle pour obtenir un produit de formule $I_A$ :

$(I_A)$

dans laquelle $Hal_1$ représente un atome d'halogène, produit sur lequel on fait agir un réactif d'halogénation puis traite éventuellement le produit obtenu par l'eau, un alcanol, un aralcanol, une amine primaire ou secondaire de formule :

$$H-N\begin{matrix}R_3\\R_4\end{matrix}$$

dans laquelle $R_3$ et $R_4$ ont la signification indiquée ci-dessus, un alkylthiol, un arylthiol ou un aralkylthiol pour obtenir un produit de formule III telle qu'indiquée ci-dessus et traite ledit produit de formule III selon la méthode indiquée pour obtenir un produit de formule IV attendu.

Les conditions opératoires et les réactifs employés dans le présent procédé sont les mêmes que ceux

employés dans les procédés ci-dessus.

L'avantage du présent procédé qui représente une des variantes d'exécution du procédé et de l'application précédents tient en ce que l'on peut opérer dans un seul récipient et sans isoler le produit intermédiaire $I_A$.

Bien entendu, aussi bien dans l'application des produits de formule I à la préparation des produits de formule IV que dans le procédé conduisant des produits de formule II aux produits de formule IV, les réactions classiques de blocage et de déblocage des groupements fonctionnels que peuvent comporter les cycles A, B, C et D peuvent être effectuées soit sur les produits de départ de formules I et II respectivement soit sur des produits intermédiaires de la synthèse.

En particulier, dans le cas où la réaction de déshydrohalogénation effectuée sur les produits intermédiaires de formule III conduit à une saponification du groupement protecteur acyle tel qu'acétyle ou formyle, on peut réacyler le produit comportant un radical hydroxyle libre à l'aide par exemple d'anhydride acétique en présence de pyridine.

L'invention a également pour objet les procédés décrits ci-dessus caractérisés en ce que l'agent de formation d'un halogénure d'acide que l'on utilise est le chlorure de thionyle.

Comme la réaction de formation de la fonction sulfine caractéristique des produits de formule I comporte de toute façon l'utilisation du chlorure de thionyle, l'enchaînement des réactifs indiqué ci-dessus, à savoir :

a/. action d'un agent de formation d'un halogénure d'acide,

b/. base tertiaire, puis

c/. chlorure de thionyle,

se résume, dans la forme préférée où l'agent de formation d'un halogénure d'acide est le chlorure de thionyle, à l'action, sur le produit de formule II du chlorure de thionyle en présence d'une base tertiaire.

L'invention a enfin pour objet, à titre de produits industriels nouveaux et notamment à titre de produits industriels nécessaires pour l'utilisation des produits de formule I, les produits de formule III :

(III)

dans laquelle A, B, C, D, R, $R_1$, $R_2$ ont la signification indiquée ci-dessus et Hal représente un atome d' halogène à l'exception d'un atome de fluor et à l'exception des produits 23,23-dichloro-N-ethylcholanamide et 3-acetoxy-23,23-dichloro-N-ethylcholanamide.

Les produits de formule II utilisés au départ du procédé de préparation des produits de formule I sont des produits connus, pour beaucoup des produits naturels de la série des acides biliaires, ou des produits qui peuvent être préparés par les méthodes usuel les à partir de ces produits naturels.

Les produits de formule IV sont des produits de la série de la progestérone. Ces produits peuvent posséder des propriétés pharmacologiques intéressantes. Par ailleurs, ces produits peuvent servir de matière de base pour la reconstruction de la chaîne désoxycortisone :

ou pour d'autres chaînes en position 17.

Les exemples suivants illustrent l'invention sans toutefois la limiter.

Exemple 1A : Acide 3 alpha formyloxy 11-oxo 23-sulfinyl 5-béta cholan 24-oïque.

On mélange 83,7 g d'acide 3-alpha formyl oxy 11-oxo 5-béta cholan 24-oïque, 840 cm3 de chlorure de

méthylène, 168 cm3 de pyridine, refroidit à +10¤C, introduit en 5 minutes environ en laissant la température remonter à +20¤C 32 cm3 de chlorure de thionyle, agite à 20¤C pendant 1 Heure, introduit en 5 minutes environ à 0¤C 84 cm3 d'eau, agite à 20¤C pendant 15 minutes, verse le mélange réactionnel dans une solution aqueuse glacée d'acide chlorhydrique, agite, décante, extrait au chlorure de méthylène, traite au charbon actif, concentre à sec par distillation sous pression réduite et obtient 98,5 g de produit attendu brut (théorie 93 g) Rf. = 0,45 en éluant avec un mélange de chloroforme d'isopropanol et d'acide acétique (85/14/1).

<u>Analyse</u> : $C_{25}H_{36}O_6S$ (464,60)

| | | | |
|---|---|---|---|
| Calculé : | C% 64,82 | H% 7,81 | S% 6,90 |
| Trouvé : | 64,8 | 7,7 | 7,0 |

<u>Spectre UV</u> (éthanol)

Max à 282 nm $E^1_1$ = 157 $\mathcal{E}$= 6 400

soit 77 % en sulfine.

Le produit est utilisé tel quel pour le stade suivant.

<u>Exemple 1B</u> : <u>acide 3 alpha hydroxy 11-oxo 23-sulfinyl 5-béta-cholan 24-oïque.</u>

L'acide 3 alpha formyloxy 11-oxo 23-sulfinyl 5-béta cholan 24-oïque obtenu en utilisant le procédé décrit à l'exemple 1A à partir de 4,18 g d'acide 3 alpha formyloxy 11-oxo 5-béta cholan 24-oïque est dissout dans un mélange de 15 cm3 de méthanol et 5 cm3 de chlorure de méthylène. On ajoute 2 cm3 d'eau, puis du carbonate de potassium jusqu'à saturation. On maintient une heure à 20¤C, verse dans un excès d'acide chlorhydrique N, extrait au chlorure de méthylène, sèche sur sulfate de sodium et distille à sec. On cristallise dans un mélange chlorure de méthylène-éther isopropylique par concentration. On obtient 1,45 g de produit attendu. F = 221¤C. Rf = 0,38 (solvant chloroforme, isopropanol, acide acétique (85/14/1).

<u>Analyse</u> : $C_{24}H_{36}O_5S$ = 436,59

| | | | |
|---|---|---|---|
| Calculé : | C% 66,02 | H% 8,31 | S% 7,34 |
| Trouvé : | 65,8 | 8,3 | 7,0 |

<u>Spectre UV</u> :

Max à 283 nm $E^1_1$ = 190 $\mathcal{E}$ = 8.300

<u>Exemple 2</u> : <u>3 alpha formyloxy 11,24-dioxo 23,23-dichloro 24-diéthylamino 5-béta cholane.</u>

On mélange 4,18 g d'acide 3 alpha formyloxy 11-oxo 5-béta cholan 24-oïque, 40 cm3 de chlorure de méthylène, 8 cm3 de pyridine, ajoute à 0¤C 1cm3 de chlorure de thionyle, puis rapidement 2,5 cm3 de chlorure de thionyle, agite à 20¤C pendant 30 minutes, introduit à 0¤C 11 cm3 de diéthylamine, agite à +5¤C pendant 30 minutes, laisse remonter à 20¤C, verse dans une solution aqueuse (1,6N) glacée d'acide chlorhydrique, extrait au chlorure de méthylène, lave avec une solution aqueuse glacée de soude, lave à l'eau, sèche la phase organique, concentre à sec sous pression réduite, chromatographie le résidu sur silice en éluant par un mélange de cyclohexane et d'acétate d'éthyle (80/20) et obtient 1,92 g de produit attendu.

<u>Contrôle</u>

Spectre IR (chloroforme) en $cm^{-1}$

Absence d'acide

présence formiate { 1 716 >C=O ; 1 198 C-O }

-C(=O)- 1 703 ep. cétone non conjugué

1 646 amide tertiaire

Spectre de RMN ($CDCl_3$) en ppm

| | | |
|---|---|---|
| H | de 18 Me | 0,7 |
| H | de 19 Me | 1,18 |
| H | de 20 Me et $CH_3$ éthyle | 1,1 à 1,24 |
| H | de $CH_2$ éthyles dédoublés | 3,34 à 3,77 |
| H | de $H_3$ | 4,8 |
| H | de CHO | 8,0 |

Exemple 3 : 3 alpha acétoxy 11,20-dioxo 5 béta pregnane.

1¤/. Déchlorhydratation :

On mélange 1,7 g de (3 alpha, 5 béta) 23,23-dichloro N,N-diéthyl 3-(formyloxy) 11-oxo cholan 24-amide, 17 cm3 de triton B, ou hydroxyde de benzyl triméthyl ammonium à 40% dans l'eau, 17 cm3 de méthanol, maintient 1 heure au reflux, refroidit, verse le mélange réactionnel dans l'eau, extrait à l'acétate d'éthyle, concentre à sec par distillation sous pression réduite et obtient 1,3 g de produit brut attendu.

2¤/. Acétylation :

On mélange 1,3 g de produit brut obtenu ci-dessus, 2,5 cm3 de pyridine, 5 cm3 d'anhydride acétique, laisse à 20¤C pendant 16 heures, refroidit à 0¤C, ajoute goutte à goutte 20 cm3 d'eau, agite, décante, extrait à l'acétate d'éthyle, concentre à sec sous pression réduite et obtient le produit acétylé utilisé tel quel pour le stade suivant.

3¤/. Ozonolyse :

On mélange le produit acétylé obtenu ci-dessus dans 20 cm3 de chlorure de méthylène avec 3 cm3 d'acide acétique, fait passer un courant d'ozone pendant 30 minutes, détruit l'ozonide en ajoutant 3 gouttes de disulfure de diméthyle, concentre à sec par distillation sous pression réduite, chromatographie le résidu sur silice (éluant : cyclohexane-acétate d'éthyle (90/10) et (cyclohexane-acétate d'éthyle 80/20) et obtient 0,370 g de produit attendu.

Spectre IR (chloroforme) en $cm^{-1}$

| | |
|---|---|
| 21 $CH_3$ | 1 363 |
| >C = O (acétate) | 1 725 |
| >C = O (11 céto) | 1 705 |

Exemple 4 : 3-alpha formyloxy 11,24-dioxo 23,23-dibromo 24-diéthylamino 5-béta cholane.

On mélange, sous atmosphère inerte, 418 g d'acide 3 alpha formyloxy 11-oxo 5 béta cholane 24-oïque, 400 cm3 de chlorure de méthylène, 80 cm3 de pyridine, introduit à 0¤C, 10 cm3 de chlorure de thionyle, puis immédiatement 16 cm3 de brome, agite à 20¤C pendant 30 minutes, ajoute à 0¤C 108 cm3 de diéthylamine, agite à +5¤C pendant 30 minutes, verse le mélange réactionnel dans une solution aqueuse glacée d'acide chlorhydrique, extrait au chlorure de méthylène, lave à l'eau, puis par une solution aqueuse N de soude glacée, puis à l'eau, sèche, traite au charbon actif, filtre, concentre à sec par distillation sous pression réduite, obtient 65,1 g deproduit brut, ajoute 1 volume d'éther isopropylique, isole par essorage le précipité formé, lave, sèche et obtient 51,6 g de produit. F = 148¤C.

On cristallise 1 g du produit obtenu dans un mélange de chlorure de méthylène et d'éther de pétrole (eb = 60 - 80¤C), essore, lave, sèche et obtient 840 mg de produit attendu pur. F = 162¤C.

Spectre IR (chloroforme) en $cm^{-1}$

Absence d'acide ; présence de formiate, cétone (1 703), amide tertiaire (1 637)

Spectre de R.M.N. en ppm

| | |
|---|---|
| H de 18 Me | 0,7 |
| H de 19 Me | 1,19 |
| H de 20 Me | 1,1-1,18 |
| $H_3$ | 4,9 |
| H de $CH_2N$ | 3,4-3,9 |
| H de $CH_O$ | 8,1 |

Exemple 5 : 3-alpha formyloxy 11,24-dioxo 23,23-dibromo 24-diéthylamino 5-béta-cholane.

On mélange, sous atmosphère inerte, 1,4 g d'acide 3 alpha-formyloxy 11-oxo 23-sulfuryl 5 béta cholan 24-oïque, 14 cm3 de chlorure de méthylène, 0,97 cm3 de pyridine, ajoute à +10¤C, 0,24 cm3 de chlorure de thionyle, agite à 20¤C pendant 10 minutes, ajoute à 0¤C 1 goutte de diterbutyle péroxyde, puis en deux minutes 0,28 cm3 de brome, agite à 20¤C pendant 1 heure, introduit à 0¤C, 1,32 cm3 de diéthylamine, agite pendant 15 minutes, verse le mélange réactionnel dans une solution aqueuse glacée d'acide chlorhydrique, extrait au chlorure méthylène, lave à l'eau, par une solution aqueuse 2 N de soude, à l'eau, concentre à sec par distillation sous pression réduite, ajoute au résidu du chlorure de méthylène et de la silice, agite pendant 30 minutes, filtre, lave, concentre à sec par distillation sous pression réduite et obtient 1,4 g de produit attendu. Par empâtage de 500 mg de ce produit dans deux volumes d'éther isopropylique, on obtient 375 mg de produit purifié utilisé tel quel pour le stade suivant.

Spectre IR (chloroforme) en $cm^{-1}$

C = O 1 700 - 1 720

C = O 1 637 lactame

Exemple 6 : 3-alpha-acétoxy 11,20-dioxo 5 béta prégnane.

1¤/. Débromhydratation :

On mélange, sous atmosphère inerte, 6,6 g de 3-alpha formyl 11,24-dioxo 23,23-dibromo 24-diéthylamino 5 béta cholane, 65 cm3 de méthanol, 65 cm3 de triton B ou hydroxyde de benzyl triméthyl ammonium en solution aqueuse de titre 43,3 g % ml), maintient pendant 1 heure au reflux, refroidit dans l'eau, extrait

au chlorure de méthylène, lave avec une solution aqueuse 0,5 M de phosphate mono sodique, à l'eau, sèche, ajoute du florisil et du charbon actif, agite, filtre, concentre à sec par distillation sous pression réduite et obtient 4,3 g de produit attendu utilisé tel quel pour le stade suivant.

2¤/. Acétylation :

On mélange sous atmosphère inerte le produit obtenu ci-dessus, 20 cm3 de pyridine, 12 cm3 d'anhydride acétique, agite à 20¤C pendant 20 heures, ajoute de l'eau, agite, verse le mélange réactionnel dans l'eau, extrait à l'acétate d'éthyle, lave à l'eau, sèche, concentre à sec par distillation sous pression réduite, entraîne le résidu de pyridine par du toluène, élimine les solvants sous pression réduite, chromatographie le résidu (1,85 g sur silice en éluant par un mélange de cyclohexane et d'acétate d'éthyle (50/50) recueille 3,17 g deproduit acétylé attendu utilisé tel quel pour le stade suivant.

3¤/. Ozonisation :

On mélange sous atmosphère inerte le produit acétylé obtenu ci-dessus, 54 cm3 de dichloroéthane, 21 cm3 d'acide acétique, amène à 0¤C, fait barboter l'ozone à +5¤C pendant 30 minutes, verse le mélange réactionnel dans l'eau, extrait au chlorure de méthylène, lave à l'eau, sèche, concentre à sec par distillation sous pression réduite, chromatographie le résidu sur silice en éluant par un mélange cyclohexane-acétate d'éthyle (75-25) et obtient 1,44 g de 3-alpha-acétoxy 11,20-dioxo 5-béta prégnane que l'on cristallise dans 4 cm3 d'éther isopropylique. On recueille 1,27 g de produit attendu pur F = 128¤C
$[alpha]_D$ = 120,5¤ (c = 1% diméthyl formamide).

Analyse: $C_{23}H_{34}O_4$ (374,41) :
Calculé : C% 73,76 H% 9,15
Trouvé : 73,79,2

Par cristallisation dans l'éther isopropylique, on obtient avec un rendement de 80% un produit. F = 135¤C.

Exemple 7 : Acide 3 alpha-formyloxy 11-oxo 23,23-dibromo 5-béta-cholan 24-oïque.

On introduit, en 5 minutes, 8 cm3 de chlorure de thionyle en laissant la température monter à 20¤C dans une solution portée à +5¤C de 20,9 g d'acide 3 alpha-formyloxy 11-oxo 5 béta-cholan 24-oïque dans 200 cm3 de chlorure ce méthylène et 32 cm3 de pyridine. On agite une heure à 20¤C, refroidit vers 10¤C et introduit en 5 minutes 8 cm3 de brome. On agite une heure vers 20¤C, verse dans un mélange eau-glace, décante, réextrait au chlorure de méthylène, sèche sur sulfate de magnésium, traite au charbon actif et distille à sec. On reprend avec 40 cm3 d'acide formique, puis chauffe 5 minutes à ébullition. On refroidit en ajoutant 40 cm3 d'éther isopropylique, glace, essore, lave à l'éther isopropylique, sèche et obtient 24,6 g de produit attendu. F = 248¤C. Rf = 0,4 (chloroforme-isopropanol-acide acétique : 85/14/1).

| Analyse : | $C_{25}H_{36}O_5Br_2$ : 576,38 | | |
|---|---|---|---|
| Calculé : | C% 52,09 | H% 6,3 | Br% 27,73 |
| Trouvé : | 52,0 | 6,3 | 27,4 |

Exemple 8 : 3-alpha hydroxy 11,20-dioxo 5-béta prégnane.

On chauffe 16 heures au reflux une suspension de 6 g de 3 alpha-formyloxy 11,24-dioxo 23,23-dibromo 24-diéthylamino 5 béta-cholane obtenu aux exemples 4 et 5 dans 60 cm3 d'éthanol et 6 cm3 de soude 10

N. On refroidit à +15¤C, verse dans 400 cm3 d'eau glacée, extrait avec 3 fois 150 cm3 d'acétate d'éthyle, lave à l'eau salée, sèche, amène à sec à 40¤C sous pression réduite. On recueille 4,04 g de produit que l'on dissout dans 200 cm3 de chlorure de méthylène et 20 cm3 de méthanol et refroidit à -65¤C sous atmosphère inerte. On fait barboter l'ozone pendant 20 minutes, met de nouveau sous atmosphère inerte et introduit à -65° C 1,9 g de phosphite de triméthyle. On agite 15 minutes à -65¤C, puis laisse revenir à 20¤C. On amène à sec à 40¤C sous pression réduite et obtient 6 g d'huile que l'on chromatographie sur silice en éluant à l'acétate d'éthyle. On recueille les fractions contenant le produit attendu et obtient 1,5 g de produit après avoir amené à sec à 40¤C. F = 168-170¤C.
$[alpha]_D$ = +100,5¤ + 3¤ c=1% acétone.

Exemple de référence : (3 alpha, 5 béta)-4-[3-(acétyloxy) 11,23-trioxo 3-24-yl] morpholine.

Stade A : Acide (3 alpha, 5 béta)-3-(acétyloxy) 11-oxo cholan 24-oique.

On mélange 200 g d'acide 3 alpha-hydroxy 11-oxo 5 béta-cholane 24-oïque, 400 cm3 d'anhydride acétique, chauffe le mélange à 45¤C, introduit en une fois 2 g d'acide paratoluène sulfanique et 20 cm3 d'acide acétique, la température s'élève vers 63¤C en 5 minutes, on maintient pendant 1 heure à 60¤C, refroidit à 55¤C, introduit en 1 heure environ 400 cm3 d'eau distillée à +55¤C, refroidit à +10¤C, essore le précipité formé, le lave, le sèche sous pression réduite et obtient 211 g de produit attendu, F = 225¤C (pureté voisine de 99¤C).

On solubilise dans le chlorure de méthylène 106 g de produit obtenu, filtre sur silice, en éluant avec un mélange de chlorure de méthylène et d'acétate d'éthyle (9/1), et obtient 105 g de produit purifié F = 225¤C.

Contrôle :

Spectre IR (chloroforme) en cm-1

OH acide

| | | |
|---|---|---|
| C=O | complexe | 1720 (ep) |
| | | 1705 (max) |
| C—O—C | acétate | 1251 |

Spectre de RMN ($CDCl_3$) en ppm

| | | | |
|---|---|---|---|
| H de $CH_3$ en 18 | 0,62- | H de ACO | 2,03 |
| H de $CH_3$ en 21 | 0,88-0,93 | H en 3 | 4,72 |
| H de $CH_3$ en 19 | 1,2 | H de COOH | 8,1 |

Stade B : (3 alpha, 5 béta)-4-[3-(acétyloxy) 11,23,24-trioxo-cholan 24-yl] morpholine.

On mélange, sous atmosphère inerte, 68 g d'acide (3 alpha, 5 béta) 3-(acétyloxy) 11-oxo-cholan 24-oïque, 250 cm3 de chlorure de méthylène, 0,35 cm3 de N, N-diméthyl formamide et ajoute au reflux du chlorure de méthylène, en 15 minutes environ, 12,8 cm3 de chlorure de thionyle. On maintient au reflux pendant 45 minutes, concentre à sec par distillation sous pression réduite, ajoute au chlorure d'acide, cristallise 250 cm3 de chlorure de méthylène et introduit à -15° C 12,8 cm3 de chlorure de thionyle. On ajoute à -25° C en 1 heure et 30 minutes environ, un mélange de 46,5 cm3 de triéthylamine et de 46,5 cm3

de chlorure de méthylène, agite la suspension obtenue à pendant 30 minutes, ajoute en maintenant la température à -25¤C en 30 minutes environ un mélange de 35,5 cm3 de morpholine et de 50 cm3 de chlorure de méthylène, agite pendant 30 minutes, ajoute en 10 minutes environ 350 cm3 d'eau en laissant remonter la température vers 0¤C. On ajoute 4,7 cm3 d'acide acétique, ajoute à +2¤/+5¤C en 1 heure 30 minutes environ 49,6 g de permanganate de potassium et dilue au cours de cette introduction par 240 cm3 d'eau et agite à +2C¤/+5¤C pendant 1 heure. On ajoute à +5¤/+10¤C en 30 minutes environ 43 g de bisulfite de sodium et simultanément une solution de 12 cm3 d'acide sulfurique concentré dans 150 cm3 d'eau glacée. On décante, lave la phase chlorométhylinique à l'eau, sèche, ajoute 5 g de sulfate de magnésium, puis 60 g d'alumine $CBT_1$ sous bonne agitation, à 20¤C, en environ 1 heure et 30 minutes. On agite encore 1 heure 30 minutes à température ambiante, filtre, concentre le filtrat à sec par distillation sous pression réduite. On ajoute au résidu 80 cm3 d'acétate d'éthyle, concentre à sec par distillation sous pression réduite pour chasser le chlorure de méthylène résiduel et ajoute au résidu 100 cm3 d'éthanol. On solubilise sous agitation vers 40¤C, refroidit à 0¤C, amorce la cristallisation, laisse au repos pendant 16 heures et isole 57,6 g de produit attendu. F = 122-123¤C.

Les liqueurs mères sont concentrées à sec, on obtient un résidu de 22 g titrant 83,5% en produit attendu.

Contrôle :

Spectre IR (chloroforme) en $cm^{-1}$

| | |
|---|---|
| morpholine N-C(=O)- | 1641 |
| région >C=O | 1723 (ep) |
| | 1715 |
| | 1704 |

Spectre de RMN ($CDCl_3$) en ppm

| | |
|---|---|
| H de $CH_3$ en 18 | 0,67 |
| H de $CH_3$ en 21 | 0,9-1,0 |
| H de $CH_3$ en 19 | 1,17 |
| H de ACO | 2,0 |
| H en 3 | 4,7 |
| H de la morpholine | 3,4-3,8 |

**Revendications**

**1.** Les produits de formule générale I :

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

$$-N\begin{matrix} R_3 \\ R_4 \end{matrix}$$

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone.

2. Les produits de formule générale I telle que définie à la revendication 1 dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7, 11 et 12 et les fonctions cétones éventuellement protégées en position 7, 11 et 12 et R représente un atome d'halogène, un radical hydroxy, un radical alkoxy ayant au plus 4 atomes de carbone ou un radical :

$$-N\begin{matrix} R'_3 \\ R'_4 \end{matrix}$$

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pépéridino, pyrrolidino ou morpholino.

3. Les produits de formule générale I telle que définie à l'une des revendications 1 ou 2 dans laquelle les noyaux A, B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un atome de chlore ou un radical hydroxyle.

4. Les produits de formule générale I telle que définie à l'une des revendications 1 à 3 répondant aux formules suivantes :
- l'acide 3 alpha-formyloxy 11-oxo 23-sulfinyl 5-béta-cholan-24-oïque.
- l'acide 3 alpha-hydroxy 11-oxo 23-sulfinyl 5-béta-cholan-24-oïque.

**5.** Procédé de préparation des produits de formule I telle que définie à la revendication 1 caractérisé en ce que l'on traite un produit de formule II :

(II)

dans laquelle A, B, C, D, $R_1$ et $R_2$ ont la signification indiquée à la revendication 1 d'abord par un agent de formation d'un halogénure d'acide, ensuite par une base tertiaire, puis par le chlorure de thionyle et enfin éventuellement par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée à la revendication 1, un alkylthiol, un arylthiol ou un aralkylthiol pour obtenir un produit de formule I attendu.

**6.** Application des produits de formule I à la préparation des produits de formule IV :

(IV)

dans laquelle A, B, C, D, $R_1$ et $R_2$ ont la signification indiquée à la revendication 1 caractérisée en ce que, lorsque R représente un radical hydroxyle, on traite éventuellement un produit de formule I par un agent de formation d'un halogénure d'acide pour obtenir un produit de formule I dans laquelle R représente un atome d'halogène et en ce que l'on traite un produit de formule I dans lequel R a la signification indiquée à la revendication 1 par un réactif d'halogénation et lorsque R représente un atome d'halogène traite éventuellement le produit intermédiaire obtenu par l'eau, un alcanol, un aralcanol, une amine primaire ou secondaire de formule :

$$-H-N\begin{matrix} R_3 \\ R_4 \end{matrix}$$

dans laquelle $R_3$ et $R_4$ ont la signification indiquée à la revendication 1, un alkylthiol, un arylthiol ou un aralkylthiol pour obtenir un produit de formule III :

(III)

produit de formule III que l'on soumet d'abord à un agent de déshydrohalogénation puis à un agent de coupure oxydante pour obtenir un produit de formule IV attendu.

7. Procédé de préparation des produits de formule IV tels que définis à la revendication 6 caractérisé en ce que l'on fait agir sur un produit de formule II un agent de formation d'un halogénure d'acide, une base tertiaire puis le chlorure de thionyle pour obtenir un produit de formule $I_A$ :

($I_A$)

dans laquelle $Hal_1$ représente un atome d'halogène, produit sur lequel on fait agir un réactif d'halogénation puis traite éventuellement le produit obtenu par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée à la revendication 1, un alkylthiol, un arylthiol ou un aralkylthiol pour obtenir un produit de formule III telle qu'indiquée à la revendication 6 et traite ledit produit de formule III selon la méthode indiquée à ladite revendication 6 pour obtenir un produit de formule IV attendu.

8. Procédé selon l'une des revendications 5 à 7 caractérisé en ce que l'agent de formation d'un halogénure d'acide que l'on utilise est le chlorure de thionyle.

9. A titre de produits industrielsnouveaux, les produits de formule III :

R2
R1
C
D
A
B
Hal
Hal
COR
(III)

dans laquelle A, B, C, D, R, $R_1$, $R_2$ ont la signification indiquée à la revendication 1 et Hal représente un atome d'halogène à l'exception d'un atome de fluor et à l'exception des produits 23,23-dichloro-N-ethylcholanamide et 3-acetoxy-23,23-dichloro-N-ethylcholanamide.

Revendications pour l'Etat contractant suivant : GR

**1.** Procédé de préparation des produits de formule générale (I) :

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

$$-N\begin{matrix} R_3 \\ R_4 \end{matrix}$$

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, caractérisé en ce que l'on traite un produit de formule (II) :

(II)

dans laquelle A, B, C, D, $R_1$ et $R_2$ ont la signification indiquée ci-dessus d'abord par un agent de formation d'un halogénure d'acide, ensuite par une base tertiaire, puis par le chlorure de thionyle et enfin éventuellement par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée ci-dessus, un alkylthiol, un arylthiol ou un aralkylthiol pour obtenir un produit de formule I attendu.

**2.** Procédé selon la revendication 1, caractérisé en ce que le produit de formule (II) utilisé au départ et les réactifs sont choisis de manière telle que l'on prépare des produits de formule (I) dans laquelle $R_1$ et

$R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7, 11 et 12 et les fonctions cétones éventuellement protégées en position 7, 11 et 12 et R représente un atome d'halogène, un radical hydroxy, un radical alkoxy ayant au plus 4 atomes de carbone ou un radical :

R'3
-N
R'4

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pépéridino, pyrrolidino ou morpholino.

**3.** Procédé selon la revendication 2, caractérisé en ce que le produit de formule (II) utilisé au départ et les réactifs sont choisis de manière telle que l'on prépare des produits de formule (I) dans laquelle les noyaux A, B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un atome de chlore ou un radical hydroxyle.

**4.** Procédé selon l'une quelconque des revendications 1 à 3, caractérisé en ce que l'on prépare les produits de formule générale (I) dont les noms suivent :

- l'acide 3 alpha-formyloxy 11-oxo 23-sulfinyl 5-béta-cholan-24-oïque.
- l'acide 3 alpha-hydroxy 11-oxo 23-sulfinyl 5-béta-cholan-24-oïque.

**5.** Application des produits de formule (I) à la préparation des produits de formule (IV) :

(IV)

dans laquelle A, B, C, D, $R_1$ et $R_2$ ont la signification indiquée à la revendication 1 caractérisée en ce que, lorsque R représente un radical hydroxyle, on traite éventuellement un produit de formule I par un agent de formation d'un halogénure d'acide pour obtenir un produit de formule I dans laquelle R représente un atome d'halogène et en ce que l'on traite un produit de formule I dans lequel R a la signification indiquée à la revendication 1 par un réactif d'halogénation et lorsque R représente un atome d'halogène traite éventuellement le produit intermédiaire obtenu par l'eau, un alcanol, un aralcanol, une amine primaire ou secondaire de formule :

$$H-N(R_3)(R_4)$$

dans laquelle $R_3$ et $R_4$ ont la signification indiquée à la revendication 1, un alkylthiol, un arylthiol ou un aralkylthiol pour obtenir un produit de formule III :

(III)

produit de formule III que l'on soumet d'abord à un agent de déshydrohalogénation puis à un agent de coupure oxydante pour obtenir un produit de formule IV attendu.

**6.** Procédé de préparation des produits de formule IV tels que définis à la revendication 5 caractérisé en ce que l'on fait agir sur un produit de formule II un agent de formation d'un halogénure d'acide, une base tertiaire puis le chlorure de thionyle pour obtenir un produit de formule $I_A$ :

R2
R1
C
D
A
B
S
O
CO-Hal1

($I_A$)

dans laquelle $Hal_1$ représente un atome d'halogène, produit sur lequel on fait agir un réactif d'halogénation puis traite éventuellement le produit obtenu par l'eau, un alcanol, un aralcanol, une amine primaire ou secondaire de formule :

dans laquelle $R_3$ et $R_4$ ont la signification indiquée à la revendication 1, un alkylthiol, un arylthiol ou un aralkylthiol pour obtenir un produit de formule III telle qu'indiquée à la revendication 6 et traite ledit produit de formule III selon la méthode indiquée à ladite revendication 6 pour obtenir un produit de formule IV attendu.

**7.** Procédé selon l'une des revendications 1, 5 ou 6, caractérisé en ce que l'agent de formation d'un halogénure d'acide que l'on utilise est le chlorure de thionyle.

**8.** A titre de produits industrielsnouveaux, les produits de formule III :

(III)

dans laquelle A, B, C, D, R, $R_1$, $R_2$ ont la signification indiquée à la revendication 1 et Hal représente un atome d'halogène à l'exception d'un atome de fluor et à l'exception des produits 23,23-dichloro-N-ethylcholanamide et 3-acetoxy-23,23-dichloro-N-ethylcholanamide.

Revendications pour l'Etat contractant suivant : ES

1. Procédé de préparation des produits de formule générale (I) :

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

$$-N(R_3)(R_4)$$

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, caractérisé en ce que l'on traite un produit de formule (II) :

(II)

dans laquelle A, B, C, D, $R_1$ et $R_2$ ont la signification indiquée ci-dessus d'abord par un agent de formation d'un halogénure d'acide, ensuite par une base tertiaire, puis par le chlorure de thionyle et enfin éventuellement par l'eau, un alcanol, un aralcanol, une amine primaire ou secondaire de formule :

$$H-N(R_3)(R_4)$$

dans laquelle $R_3$ et $R_4$ ont la signification indiquée ci-dessus, un alkylthiol, un arylthiol ou un aralkylthiol pour obtenir un produit de formule I attendu.

2. Procédé selon la revendication 1, caractérisé en ce que le produit de formule (II) utilisé au départ et les réactifs sont choisis de manière telle que l'on prépare des produits de formule (I) dans laquelle $R_1$ et

$R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7, 11 et 12 et les fonctions cétones éventuellement protégées en position 7, 11 et 12 et R représente un atome d'halogène, un radical hydroxy, un radical alkoxy ayant au plus 4 atomes de carbone ou un radical :

$$-N\begin{matrix} R'_3 \\ R'_4 \end{matrix}$$

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pépéridino, pyrrolidino ou morpholino.

3. Procédé selon la revendication 2, caractérisé en ce que le produit de formule (II) utilisé au départ et les réactifs sont choisis de manière telle que l'on prépare des produits de formule (I) dans laquelle les noyaux A, B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un atome de chlore ou un radical hydroxyle.

4. Application des produits de formule (I) à la préparation des produits de formule (IV) :

R2, R1, A, B, C, D, O

(IV)

dans laquelle A, B, C, D, $R_1$ et $R_2$ ont la signification indiquée à la revendication 1 caractérisée en ce que, lorsque R représente un radical hydroxyle, on traite éventuellement un produit de formule I par un agent de formation d'un halogénure d'acide pour obtenir un produit de formule I dans laquelle R représente un atome d'halogène et en ce que l'on traite un produit de formule I dans lequel R a la signification indiquée à la revendication 1 par un réactif d'halogénation et lorsque R représente un atome d'halogène traite éventuellement le produit intermédiaire obtenu par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée à la revendication 1, un alkylthiol, un arylthiol ou un aralkylthiol pour obtenir un produit de formule III :

R2, R1, A, B, C, D, Hal, Hal, COR

(III)

produit de formule III que l'on soumet d'abord à un agent de déshydrohalogénation puis à un agent de coupure oxydante pour obtenir un produit de formule IV attendu.

5. Procédé de préparation des produits de formule IV tels que définis à la revendication 4 caractérisé en ce que l'on fait agir sur un produit de formule II un agent de formation d'un halogénure d'acide, une base tertiaire puis le chlorure de thionyle pour obtenir un produit de formule $I_A$ :

(I_A)

dans laquelle $Hal_1$ représente un atome d'halogène, produit sur lequel on fait agir un réactif d'halogénation puis traite éventuellement le produit obtenu par l'eau, un alcanol, un aralcanol, une amine primaire ou secondaire de formule :

$$H-N\begin{matrix} R_3 \\ R_4 \end{matrix}$$

dans laquelle $R_3$ et $R_4$ ont la signification indiquée à la revendication 1, un alkylthiol, un arylthiol ou un aralkylthiol pour obtenir un produit de formule III telle qu'indiquée à la revendication 6 et traite ledit produit de formule III selon la méthode indiquée à ladite revendication 4 pour obtenir un produit de formule IV attendu.

6. Procédé selon l'une des revendications 1, 4 ou 5, caractérisé en ce que l'agent de formation d'un halogénure d'acide que l'on utilise est le chlorure de thionyle.

## Claims

1. The products of general formula (I) :

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a

R3
-N
R4

radical in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms.

2. The products of general formula (1) as defined in claim 1 in which $R_1$ and $R_2$ each represent a methyl radical and the nuclei A, B, C, D carry in position 3 an optionally protected hydroxyl function and, optionally, one or more other functions chosen from the hydroxyl functions optionally protected in position 6, 7, 11 and 12 and the ketone functions optionally protected in position 7, 11 and 12 and R represents a halogen atom, a hydroxy radical, an alkoxy radical having at most 4 carbon atoms or a radical:

$$N\begin{matrix} R'_3 \\ R'_4 \end{matrix}$$

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

3. The products of general formula (I) as defined in any one of claims 1 or 2 in which the nuclei A, B, C, D carry in position 3 an optionally protected hydroxyl radical and optionally one or more functions chosen from the hydroxyl functions optionally protected in position 12 and the ketone functions optionally protected in position 11 or 12 and R represents a chlorine atom or a hydroxyl radical.

4. The products of general formula (1) as defined in one of claims 1 to 3 corresponding to the following formulae:
   - 3-alpha-formyloxy-11-oxo-23-sulphinyl-5-beta-cholan-24-oic acid.
   - 3-alpha-hydroxy-11-oxo-23-sulphinyl-5-beta-cholan-24-oic acid.

5. Preparation process for the products of formula (I) as defined in claim 1 characterized in that a product of formula (II):

R2
R1
C
D
A
B
CO2H
(II)

in which A, B, C, D, $R_1$ and $R_2$ have the meaning indicated in claim 1 is first treated with an acid halide formation agent, then by a tertiary base, then by thionyl chloride and finally, optionally, by water, an alkanol, an aralkanol, a primary or secondary amine of formula:

$$H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a desired product of formula (I).

6. The use of products of formula (I) in the preparation of products of formula (IV):

R2
R1
C
D
A
B
O
(IV)

in which A, B, C, D, $R_1$ and $R_2$ have the meaning indicated in claim 1 characterized in that, when $R_1$ represents a hydroxyl radical, a product of formula (I) is optionally treated with an acid halide formation agent in order to obtain a product of formula (I) in which R represents a halogen atom and in that a product of formula (I) in which R has the meaning indicated in claim 1 is treated with a halogenation reagent and when R represents a halogen atom the intermediate product obtained is optionaly treated with water, an alkanol, an aralkanol, a primary or secondary amine of formula:

$$-H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a product of formula (III):

(III)

which product of formula (III) is first subjected to a dehydro-halogenation agent, then to an oxydizing cleavage agent in order to obtain an expected product of formula (IV).

7. Preparation process for products of formula (IV) as defined in claim 6 characterized in that an acid halide formation agent, a tertiary base, followed by thionyl chloride are reacted on a product of formula (II) in order to obtain a product of formula ($I_A$):

(I$_A$)

in which $Hal_1$ represents a halogen atom, on which product a halogenation agent is reacted then the obtained product is optionally treated with water, an alkanol, an aralkanol, a primary or secondary amine of formula:

$$H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a product of formula (III) as indicated in claim 6 and said product of formula (III) is treated according to the method indicated in said claim 6 in order to obtain an expected product of formula (IV).

8. Process according to one of claims 5 to 7 characterized in that the acid halide formation agent which is used is thionyl chloride.

9. As new industrial products, the products of formula (III):

(III)

in which A, B, C, D, R, $R_1$, $R_2$ have the meaning indicated in claim 1 and Hal represents a halogen atom with the exception of a fluorine atom and with the exception of the products 23,23-dichloro-N-ethylcholanamide and 3-acetoxy-23,23-dichloro-N-ethylcholanamide.

Claims for the following Contracting State : GR

1. Preparation process for the products of general formula (I) :

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl

radical, an alkoxy radical having 1 to 6 carbon atoms, an aralkoxy radical having 7 to 15 carbon atoms, a

-N
R3
R4

radical in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms, characterized in that a product of formula (II):

R1
R2
A
B
C
D
CO2H
(II)

in which A, B, C, D, $R_1$ and $R_2$ have the meaning indicated above is first treated with an acid halide formation agent, then with a tertiary base, then with thionyl chloride and finally, optionally, with water, an alkanol, an aralkanol, a primary or secondary amine of formula:

$$H-N\begin{matrix} R_3 \\ R_4 \end{matrix}$$

in which $R_3$ and $R_4$ have the meaning indicated in claim 1, an alkylthiol, an arylthiol or an aralkylthiol, in order to obtain an expected product of formula (I).

**2.** Process according to claim 1, characterized in that the product of formula (II) used at the start and the reagents are chosen in such a manner so that the products of formula (I) are prepared in which $R_1$ and $R_2$ each represent a methyl radical and the nuclei A, B, C, D carry in position 3 an optionally protected hydroxyl function and, optionally, one or more other functions chosen from the hydroxyl functions optionally protected in position 6, 7, 11 and 12 and the ketone functions optionally protected in position 7, 11 and 12 and R represents a halogen atom, a hydroxy radical, an alkoxy radical having at most 4 carbon atoms or a radical:

$$N\begin{matrix} R'_3 \\ R'_4 \end{matrix}$$

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

**3.** Process according to claim 2, characterized in that the product of formula (II) used at the start and the reagents are chosen in such a way so that the products of formula (I) are prepared in which the nuclei

A, B, C, D carry in position 3 an optionally protected hydroxyl radical and optionally one or more functions chosen from the hydroxyl functions optionally protected in position 12 and the ketone functions optionally protected in position 11 or 12 and R represents a chlorine atom or a hydroxyl radical.

4. Process according to any one of claims 1 to 3, characterized in that the products of general formula (I) are prepared of which the names follow:
   - 3-alpha-formyloxy-11-oxo-23-sulphinyl-5-beta-cholan-24-oic acid.
   - 3-alpha-hydroxy-11-oxo-23-sulphinyl-5-beta-cholan-24-oic acid.

5. The use of products of formula (I) in the preparation of products of formula (IV):

(IV)

in which A, B, C, D, $R_1$ and $R_2$ have the meaning indicated in claim 1 characterized in that, when R represents a hydroxyl radical, a product of formula (I) is optionally treated with an acid halide formation agent in order to obtain a product of formula (I) in which R represents a halogen atom and in that a product of formula (I) in which R has the meaning indicated in claim 1 is treated by a halogenation reagent and when R represents a halogen atom the intermediate product obtained is treated with water, an alkanol, an aralkanol, a primary or secondary amine of formula:

$$-H-N\begin{matrix} R_3 \\ R_4 \end{matrix}$$

in which $R_3$ and $R_4$ have the meaning indicated in claim 1, an alkylthiol, an arylthiol or an aralkylthiol, in order to obtain a product of formula (III):

(III)

which product of formula (III) is first subjected to a dehydro-halogenation agent, then to an oxydizing cleavage agent in order to obtain an expected product of formula (IV).

6. Preparation process for products of formula (IV) as defined in claim 5 characterized in that an acid halide formation agent, a tertiary base, followed by thionyl chloride are reacted on a product of formula (II) in order to obtain a product of formula ($I_A$):

$$\text{(I}_A\text{)}$$

in which $Hal_1$ represents a halogen atom, on which product a halogenation agent is reacted then the obtained product is optionally treated with water, an alkanol, an aralkanol, a primary or secondary amine of formula:

$$H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a product of formula (III) as indicated in claim 6 and said product of formula (III) is treated according to the method indicated in said claim 6 in order to obtain a desired product of formula (IV).

7. Process according to one of claims 1, 5 or 6 characterized in that the acid halide formation agent which is used is thionyl chloride.

8. As new industrial products, the products of formula (III):

(III)

in which A, B, C, D, R, $R_1$, $R_2$ have the meaning indicated in claim 1 and Hal represents a halogen atom with the exception of a fluorine atom and with the exception of the products 23,23-dichloro-N-ethylcholanamide and 3-acetoxy-23,23-dichloro-N-ethylcholanamide.

Claims for the following Contracting State : ES

1. Preparation process for the products of general formula (I) :

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or

more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a

$$-N\begin{matrix}\diagup R_3 \\ \diagdown R_4\end{matrix}$$

radical in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms, characterized in that a product of formula (II):

R1 R2 A B C D $CO_2H$ (II)

in which A, B, C, D, $R_1$ and $R_2$ have the meaning indicated in above is first treated with an acid halide formation agent, then by a tertiary base, then by thionyl chloride and finally, optionally, by water, an alkanol, an aralkanol, a primary or secondary amine of formula:

$$H-N\begin{matrix}\diagup R_3 \\ \diagdown R_4\end{matrix}$$

in which $R_3$ and $R_4$ have the meaning indicated in claim 1, an alkylthiol, an arylthiol or an aralkylthiol in order to obtain an expected product of formula (I).

2. Process according to claim 1, characterized in that the product of formula (II) used at the start and the reagents are chosen in such a manner so that the products of formula (I) are prepared in which $R_1$ and $R_2$ each represent a methyl radical and the nuclei A, B, C, D carry in position 3 an optionally protected hydroxyl function and, optionally, one or more other functions chosen from the hydroxyl functions optionally protected in position 6, 7, 11 and 12 and the ketone functions optionally protected in position 7, 11 and 12 and R represents a halogen atom, a hydroxy radical, an alkoxy radical having at most 4 carbon atoms or a radical:

R'3
N
R'4

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

3. Process according to claim 2, characterized in that the product of formula (II) used at the start and the reagents are chosen in such a way so that the products of formula (I) are prepared in which the nuclei

A, B, C, D carry in position 3 an optionally protected hydroxyl radical and optionally one or more functions chosen from the hydroxyl functions optionally protected in position 12 and the ketone functions optionally protected in position 11 or 12 and R represents a chlorine atom or a hydroxyl radical.

**4.** The use of the products of formula (I) in the preparation of products of formula (IV):

(IV)

in which A, B, C, D, $R_1$ and $R_2$ have the meaning indicated in claim 1 characterized in that, when R represents a hydroxyl radical, a product of formula (I) is optionally treated with an acid halide formation agent in order to obtain a product of formula (I) in which R represents a halogen atom and in that a product of formula (I) in which R has the meaning indicated in claim 1 is treated with a halogenation reagent and when R represents a halogen atom the intermediate product obtained is treated with water, an alkanol, an aralkanol, a primary or secondary amine of formula:

$$-H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a product of formula (III):

(III)

which product of formula (III) is first subjected to a dehydro-halogenation agent, then to an oxydizing cleavage agent in order to obtain an expected product of formula (IV).

**5.** Preparation process for products of formula (IV) as defined in claim 4, characterized in that an acid halide formation agent, a tertiary base, followed by thionyl chloride are reacted on a product of formula (II) in order to obtain a product of formula ($I_A$):

($I_A$)

in which $Hal_1$ represents a halogen atom, on which product a halogenation agent is reacted then the

obtained product is optionally treated with water, an alkanol, an aralkanol, a primary or secondary amine of formula:

$$H-N\begin{matrix} R_3 \\ R_4 \end{matrix}$$

in which $R_3$ and $R_4$ have the meaning indicated in claim 1, an alkylthiol, an arylthiol or an aralkylthiol in order to obtain a product of formula (III) as indicated in claim 6 and said product of formula (III) is treated according to the method indicated in said claim 6 in order to obtain an expected product of formula (IV).

6. Process according to one of claims 1, 4 or 5 characterized in that the acid halide formation agent which is used is thionyl chloride.

**Ansprüche**

1. Verbindungen der allgemeinen Formel I:

(I)

worin $R_1$ ein Wasserstoffatom oder eine Methylgruppe bedeutet, $R_2$ eine Methyl- oder Ethylgruppe bedeutet, die Ringe A, B, C, D gegebenenfalls eine oder mehrere Doppelbindungen aufweisen und gegebenenfalls substituiert sind durch eine oder mehrere gegebenenfalls geschützte Hydroxyl- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen, eine Gruppe:

R3
-N
R4

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet.

2. Verbindungen der allgemeinen Formel I gemäß Anspruch 1, worin $R_1$ und $R_2$ jeweils eine Methylgruppe bedeuten, und die Ringe A, B, C, D in 3-Position eine gegebenenfalls geschützte Hydroxyfunktion und gegebenenfalls eine oder mehrere andere Funktionen aufweisen, ausgewählt aus gegebenenfalls geschützten Hydroxyfunktionen in 6-, 7-, 11- und 12-Position und gegebenenfalls geschützten Ketofunktionen in 7-, 11- und 12-Position, und worin R ein Halogenatom, eine Hydroxygruppe, eine Alkoxygruppe mit höchstens 4 Kohlenstoffatomen oder eine Gruppe:

bedeutet, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bilden.

3. Verbindungen der allgemeinen Formel I gemäß einem der Ansprüche 1 oder 2, worin die Ringe A, B, C, D in 3-Position eine gegebenenfalls geschützte Hydroxygruppe und gegebenenfalls eine oder mehrere weitere Funktionen aufweisen, ausgewählt aus gegebenenfalls geschützten Hydroxyfunktionen in 12-Position und gegebenenfalls geschützten Ketofunktionen in 11- oder 12-Position, und worin R ein Chloratom oder eine Hydroxygruppe bedeutet.

4. Verbindungen der allgemeinen Formel I gemäß einem der Ansprüche 1 bis 3 entsprechend den folgenden Formeln:
   - 3α-Formyloxy-11-oxo-23-sulfinyl-5β-cholan-24-säure,
   - 3α-Hydroxy-11-oxo-23-sulfinyl-5β-cholan-24-säure.

5. Verfahren zur Herstellung von Verbindungen der Formel I gemäß Anspruch 1, dadurch gekennzeichnet, daß man eine Verbindung der Formel II:

worin A, B, C, D, $R_1$ und $R_2$ die in Anspruch 1 angegebene Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

worin $R_3$ und $R_4$ die in Anspruch 1 angegebene Bedeutung besitzen, einem Alkylthiol, einem Arylthiol oder einem Aralkylthiol behandelt, um eine gewünschte Verbindung der Formel I zu erhalten.

6. Verwendung der Verbindungen der Formel I zur Herstellung von Verbindungen der Formel IV:

worin A, B, C, D, $R_1$ und $R_2$ die in Anspruch 1 angegebene Bedeutung besitzen, dadurch gekennzeichnet, daß, wenn R eine Hydroxygruppe bedeutet, man gegebenenfalls eine Verbindung der Formel I mit einem ein Säurehalogenid bildenden Reagens behandelt, um eine Verbindung der Formel I zu erhalten, worin R ein Halogenatom bedeutet, und daß man eine Verbindung der Formel I, worin R die in Anspruch 1 angegebene Bedeutung besitzt, mit einem Halogenierungsreagens behandelt, und daß, wenn R ein Halogenatom bedeutet, man gegebenenfalls das erhaltene Zwischenprodukt mit Wasser, einem Alkanol, einem Aralkanol, einem primären oder sekundären Amin der Formel:

$$H-N(R_3)(R_4)$$

worin $R_3$ und $R_4$ die in Anspruch 1 angegebene Bedeutung besitzen, einem Alkylthiol, einem Arylthiol oder einem Aralkylthiol behandelt, um eine Verbindung der Formel III:

(III)

zu erhalten, und daß man die Verbindung der Formel III zunächst einem Reagens zur Halogenwasserstoffabspaltung und sodann einem Reagens zur oxidierenden Spaltung unterwirft, um eine gewünschte Verbindung der Formel IV zu erhalten.

7. Verfahren zur Herstellung der Verbindungen der Formel IV, wie in Anspruch 6 definiert, dadurch gekennzeichnet, daß man auf eine Verbindung der Formel II ein ein Säurehalogenidbildendes Reagens, eine tertiäre Base, dann Thionylchlorid einwirken läßt, um eine Verbindung der Formel $I_A$:

($I_A$)

zu erhalten, worin $Hal_1$ ein Halogenatom bedeutet, auf diese Verbindung ein Halogenierungsreagens einwirken läßt, die erhaltene Verbindung sodann gegebenenfalls mit Wasser, einem Alkanol, einem Aralkanol, einem primären oder sekundären Amin der Formel:

R3
H-N
R4

worin $R_3$ und $R_4$ die in Anspruch 1 angegebene Bedeutung besitzen, einem Alkylthiol, einem Arylthiol oder einem Aralkylthiol behandelt, um eine Verbindung der Formel III, wie in Anspruch 6 angegeben, zu erhalten, und daß man diese Verbindung der Formel III gemäß der im genannten Anspruch 6 angegebenen Methode behandelt, um eine gewünschte Verbindung der Formel IV zu erhalten.

8. Verfahren nach einem der Ansprüche 5 bis 7, dadurch gekennzeichnet, daß das verwendete, ein

Säurehalogenid bildende Reagens Thionylchlorid ist.

9. Verbindungen der Formel III:

(III)

als neue industrielle Verbindungen, worin A, B, C, D, R, $R_1$ und $R_2$ die im Anspruch 1 angegebene Bedeutung besitzen, und Hal ein Halogenatom, ausgenommen ein Fluoratom, bedeutet, mit Ausnahme der Verbindungen 23,23-Dichlor-N-ethyl-cholanamid und 3-Acetoxy-23,23-dichlor-N-ethyl-cholanamid.

Patentansprüche für folgenden Vertragsstaat : GR

1. Verfahren zur Herstellung von Verbindungen der allgemeinen Formel I:

R2
R1
C
D
A
B
COR
S
O

(I)

worin $R_1$ ein Wasserstoffatom oder eine Methylgruppe bedeutet, $R_2$ eine Methyl- oder Ethylgruppe bedeutet, die Ringe A, B, C, D gegebenenfalls eine oder mehrere Doppelbindungen aufweisen und gegebenenfalls substituiert sind durch eine oder mehrere gegebenenfalls geschützte Hydroxyl- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen,
eine Gruppe:

$$-N\begin{matrix} R_3 \\ R_4 \end{matrix}$$

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, dadurch gekennzeichnet, daß man eine Verbindung der Formel II:

R2
R1
C
D
A
B
CO2H

(II)

worin A, B, C, D, $R_1$ und $R_2$ die vorstehend angegebene Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

$$H-N\begin{matrix}R_3\\R_4\end{matrix}$$

worin $R_3$ und $R_4$ die vorstehend angegebene Bedeutung besitzen, einem Alkylthiol, einem Arylthiol oder einem Aralkylthiol behandelt, um eine gewünschte Verbindung der Formel I zu erhalten.

2. Verfahren nach Anspruch 1, dadurch gekennzeichnet, daß die verwendete Ausgangsverbindung der Formel II und die Reaktionspartner derart gewählt sind, daß man Verbindungen der Formel I herstellt, worin $R_1$ und $R_2$ jeweils eine Methylgruppe bedeuten, und die Ringe A, B, C, D in 3-Position eine gegebenenfalls geschützte Hydroxyfunktion und gegebenenfalls eine oder mehrere andere Funktionen aufweisen, ausgewählt aus gegebenenfalls geschützten Hydroxyfunktionen in 6-, 7-, 11- und 12-Position und gegebenenfalls geschützten Ketofunktionen in 7-, 11- und 12-Position, und worin R ein Halogenatom, eine Hydroxygruppe, eine Alkoxygruppe mit höchstens 4 Kohlenstoffatomen oder eine Gruppe:

-N
R'3
R'4

bedeutet, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bilden.

3. Verfahren gemäß Anspruch 2, dadurch gekennzeichnet, daß man die verwendete Ausgangsverbindung der Formel II und die Reaktionspartner derart wählt, daß man Verbindungen der Formel I herstellt, worin die Ringe A, B, C, D in 3-Position eine gegebenenfalls geschützte Hydroxygruppe und gegebenenfalls eine oder mehrere weitere Funktionen aufweisen, ausgewählt aus gegebenenfalls geschützten Hydroxyfunktionen in 12-Position und gegebenenfalls geschützten Ketofunktionen in 11- oder 12-Position, und worin R ein Chloratom oder eine Hydroxygruppe bedeutet.

4. Verfahren gemäß einem der Ansprüche 1 bis 3, dadurch gekennzeichnet, daß man Verbindungen der allgemeinen Formel I mit folgenden Namen herstellt:
   - 3α-Formyloxy-11-oxo-23-sulfinyl-5β-cholan-24-säure,
   - 3α-Hydroxy-11-oxo-23-sulfinyl-5β-cholan-24-säure.

5. Verwendung der Verbindungen der Formel I zur Herstellung von Verbindungen der Formel IV:

R2
R1
O
A
B
C
D
(IV)

worin A, B, C, D, $R_1$ und $R_2$ die in Anspruch 1 angegebene Bedeutung besitzen, dadurch gekennzeichnet, daß, wenn R eine Hydroxygruppe bedeutet, man gegebenenfalls eine Verbindung der Formel I mit

einem ein Säurehalogenid bildenden Reagens behandelt, um eine Verbindung der Formel I zu erhalten, worin R ein Halogenatom bedeutet, und daß man eine Verbindung der Formel I, worin R die in Anspruch 1 angegebene Bedeutung besitzt, mit einem Halogenierungsreagens behandelt, und daß, wenn R ein Halogenatom bedeutet, man gegebenenfalls das erhaltene Zwischenprodukt mit Wasser, einem Alkanol, einem Aralkanol, einem primären oder sekundären Amin der Formel:

$$H-N(R_3)(R_4)$$

worin $R_3$ und $R_4$ die in Anspruch 1 angegebene Bedeutung besitzen, einem Alkylthiol, einem Arylthiol oder einem Aralkylthiol behandelt, um eine Verbindung der Formel III:

(III)

zu erhalten, und daß man die Verbindung der Formel III zunächst einem Reagens zur Halogenwasserstoffabspaltung und sodann einem Reagens zur oxidierenden Spaltung unterwirft, um eine gewünschte Verbindung der Formel IV zu erhalten.

6. Verfahren zur Herstellung von Verbindungen der Formel IV, wie sie in Anspruch 5 definiert sind, dadurch gekennzeichnet, daß man auf eine Verbindung der Formel II ein ein Säurehalogenid bildendes Reagens, eine tertiäre Base, dann Thionylchlorid einwirken läßt, um eine Verbindung der Formel $I_A$:

($I_A$)

zu erhalten, worin $Hal_1$ ein Halogenatom bedeutet, auf diese Verbindung ein Halogenierungsreagens einwirken läßt, die erhaltene Verbindung sodann gegebenenfalls mit Wasser, einem Alkanol, einem Aralkanol, einem primären oder sekundären Amin der Formel:

$$H-N(R_3)(R_4)$$

worin $R_3$ und $R_4$ die in Anspruch 1 angegebene Bedeutung besitzen, einem Alkylthiol, einem Arylthiol oder einem Aralkylthiol behandelt, um eine Verbindung der Formel III, wie in Anspruch 5 angegeben, zu erhalten, und daß man diese Verbindung der Formel III gemäß der im genannten Anspruch 5 angegebenen Methode behandelt, um eine gewünschte Verbindung der Formel IV zu erhalten.

7. Verfahren nach einem der Ansprüche 1, 5 oder 6, dadurch gekennzeichnet, daß das verwendete, ein Säurehalogenid bildende Reagens Thionylchlorid ist.

**8.** Verbindungen der Formel III:

(III)

als neue industrielle Verbindungen, worin A, B, C, D, R, $R_1$ und $R_2$ die im Anspruch 1 angegebene Bedeutung besitzen, und Hal ein Halogenatom, ausgenommen ein Fluoratom, bedeutet, mit Ausnahme der Verbindungen 23,23-Dichlor-N-ethyl-cholanamid und 3-Acetoxy-23,23-dichlor-N-ethyl-cholanamid.

Patentansprüche für folgenden Vertragsstaat : ES

**1.** Verfahren zur Herstellung von Verbindungen der allgemeinen Formel I:

(I)

worin $R_1$ ein Wasserstoffatom oder eine Methylgruppe bedeutet, $R_2$ eine Methyl- oder Ethylgruppe bedeutet, die Ringe A, B, C, D gegebenenfalls eine oder mehrere Doppelbindungen aufweisen und gegebenenfalls substituiert sind durch eine oder mehrere gegebenenfalls geschützte Hydroxyl- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen,
eine Gruppe:

$$-N\begin{matrix} R_3 \\ R_4 \end{matrix}$$

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, dadurch gekennzeichnet, daß man eine Verbindung der Formel II:

R2
R1
C
D
A
B
CO2H
(II)

worin A, B, C, D, $R_1$ und $R_2$ die vorstehend angegebene Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

$$H{-}N(R_3)(R_4)$$

worin $R_3$ und $R_4$ die vorstehend angegebene Bedeutung besitzen, einem Alkylthiol, einem Arylthiol oder einem Aralkylthiol behandelt, um eine gewünschte Verbindung der Formel I zu erhalten.

2. Verfahren nach Anspruch 1, dadurch gekennzeichnet, daß die verwendete Ausgangsverbindung der Formel II und die Reaktionspartner derart gewählt sind, daß man Verbindungen der Formel I herstellt, worin $R_1$ und $R_2$ jeweils eine Methylgruppe bedeuten, und die Ringe A, B, C, D in 3-Position eine gegebenenfalls geschützte Hydroxyfunktion und gegebenenfalls eine oder mehrere andere Funktionen aufweisen, ausgewählt aus gegebenenfalls geschützten Hydroxyfunktionen in 6-, 7-, 11- und 12-Position und gegebenenfalls geschützten Ketofunktionen in 7-, 11- und 12-Position, und worin R ein Halogenatom, eine Hydroxygruppe, eine Alkoxygruppe mit höchstens 4 Kohlenstoffatomen oder eine Gruppe:

$$-N(R'_3)(R'_4)$$

bedeutet, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bilden.

3. Verfahren gemäß Anspruch 2, dadurch gekennzeichnet, daß man die verwendete Ausgangsverbindung der Formel II und die Reaktionspartner derart wählt, daß man Verbindungen der Formel I herstellt, worin die Ringe A, B, C, D in 3-Position eine gegebenenfalls geschützte Hydroxygruppe und gegebenenfalls eine oder mehrere weitere Funktionen aufweisen, ausgewählt aus gegebenenfalls geschützten Hydroxyfunktionen in 12-Position und gegebenenfalls geschützten Ketofunktionen in 11- oder 12-Position, und worin R ein Chloratom oder eine Hydroxygruppe bedeutet.

4. Verwendung der Verbindungen der Formel I zur Herstellung von Verbindungen der Formel IV:

R2
R1
A
B
C
D
O

(IV)

worin A, B, C, D, $R_1$ und $R_2$ die in Anspruch 1 angegebene Bedeutung besitzen, dadurch gekennzeichnet, daß, wenn R eine Hydroxygruppe bedeutet, man gegebenenfalls eine Verbindung der Formel I mit einem ein Säurehalogenid bildenden Reagens behandelt, um eine Verbindung der Formel I zu erhalten, worin R ein Halogenatom bedeutet, und daß man eine Verbindung der Formel I, worin R die in Anspruch 1 angegebene Bedeutung besitzt, mit einem Halogenierungsreagens behandelt, und daß, wenn R ein Halogenatom bedeutet, man gegebenenfalls das erhaltene Zwischenprodukt mit Wasser, einem Alkanol, einem Aralkanol, einem primären oder sekundären Amin der Formel:

$$H-N\begin{matrix}R_3\\ R_4\end{matrix}$$

worin $R_3$ und $R_4$ die in Anspruch 1 angegebene Bedeutung besitzen, einem Alkylthiol, einem Arylthiol oder einem Aralkylthiol behandelt, um eine Verbindung der Formel III:

R2
R1
C
D
A
B
Hal
Hal
O=C
R

(III)

zu erhalten, und daß man die Verbindung der Formel III zunächst einem Reagens zur Halogenwasserstoffabspaltung und sodann einem Reagens zur oxidierenden Spaltung unterwirft, um eine gewünschte Verbindung der Formel IV zu erhalten.

5. Verfahren zur Herstellung der Verbindungen der Formel IV, wie in Anspruch 4 definiert, dadurch gekennzeichnet, daß man auf eine Verbindung der Formel II ein ein Säurehalogenid bildendes Reagens, eine tertiäre Base, dann Thionylchlorid einwirken läßt, um eine Verbindung der Formel $I_A$:

$(I_A)$

zu erhalten, worin $Hal_1$ ein Halogenatom bedeutet, auf diese Verbindung ein Halogenierungsreagens einwirken läßt, die erhaltene Verbindung sodann gegebenenfalls mit Wasser, einem Alkanol, einem Aralkanol, einem primären oder sekundären Amin der Formel:

$$H-N\begin{matrix}R_3\\ R_4\end{matrix}$$

worin $R_3$ und $R_4$ die in Anspruch 1 angegebene Bedeutung besitzen, einem Alkylthiol, einem Arylthiol oder einem Aralkylthiol behandelt, um eine Verbindung der Formel III, wie in Anspruch 4 angegeben, zu erhalten, und daß man diese Verbindung der Formel III gemäß der im genannten Anspruch 4 angegebenen Methode behandelt, um eine gewünschte Verbindung der Formel IV zu erhalten.

6. Verfahren nach einem der Ansprüche 1, 4 oder 5, dadurch gekennzeichnet, daß das verwendete, ein Säurehalogenid bildende Reagens Thionylchlorid ist.